(19) Europäisches Patentamt / European Patent Office / Office européen des brevets

(11) **EP 4 198 054 A1**

(12) **EUROPEAN PATENT APPLICATION**
published in accordance with Art. 153(4) EPC

(43) Date of publication:
**21.06.2023 Bulletin 2023/25**

(51) International Patent Classification (IPC):
**C07K 16/18** (2006.01)   **C12N 15/13** (2006.01)
**A61K 39/395** (2006.01)   **A61P 35/00** (2006.01)

(21) Application number: **22822840.9**

(22) Date of filing: **13.10.2022**

(86) International application number:
**PCT/CN2022/125196**

(87) International publication number:
**WO 2023/071821 (04.05.2023 Gazette 2023/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.10.2021   CN 202111263981**

(71) Applicant: **Nanjing Anji Biological Technology Co., Ltd.
Nanjing, Jiangsu 210033 (CN)**

(72) Inventor: **XU, Hanmei
Nanjing, Jiangsu 210033 (CN)**

(74) Representative: **Wichmann, Hendrik
Wuesthoff & Wuesthoff
Patentanwälte PartG mbB
Schweigerstraße 2
81541 München (DE)**

Remarks:
The complete document including Reference Table(s) and the Sequence Listing(s) can be downloaded from the EPO website

(54) **ANTI-HMMW ANTIBODY, COMPOSITION CONTAINING SAME, NUCLEIC ACID MOLECULE ENCODING SAME, AND USE THEREOF**

(57)    The present disclosure relates to an anti-HMMW antibody, a composition comprising the antibody, and a nucleic acid molecule encoding the antibody, and use of the antibody, the composition or the nucleic acid molecule.

Fig. 5

**Description**

**TECHNICAL FIELD**

The present disclosure relates to an anti-HMMW antibody, a composition comprising the antibody, and a nucleic acid molecule encoding the antibody, and use of the antibody, the composition or the nucleic acid molecule.

**BACKGROUND ART**

The part of statements merely provides background information related to the present disclosure and do not necessarily constitute the prior art.

Long non-coding RNAs (lncRNAs) are a class of RNA molecules with transcripts longer than 200 nt. It is generally considered that lncRNA does not encode proteins, but interacts with proteins, DNA and RNA in the form of RNA to regulate gene expression in terms of epigenetic modification (chromatin remodeling and modification, and DNA/RNA methylation), transcription level (binding transcription factors), and post-transcriptional level (RNA cleavage, mRNA stabilization and translation, and as a ceRNA). In 2011, scientists have surprisingly found that lncRNA may translate small short peptides by deep sequencing of ribosome-protected fragments. However, limited by depth and accuracy of transcriptome sequencing techniques and analytical methods, there is a great debate about non-coding RNA containing an open reading frame with coding capability at the international level. In 2015, the team of Eric Olson professor of University of Texas Southwestern Medical Center found an lncRNA specifically expressed in skeletal muscle. The lncRNA could encode a 46-amino acid micropeptide myoregulin (MLN) which was found to play an important role in the regulation of calcium homeostasis of myocardial cells. In 2016, Eric Olson laboratories reported in Science another micropeptide DWORF encoded by lncRNA, wherein the micropeptide was highly expressed in mouse heart and could regulate muscle contraction. In recent years, scientists all over the world have continued to discover that the micropeptides encoded by lncRNA mainly participate in important physiological or pathological processes such as regulation of muscle formation, amino acid metabolism, mRNA post-transcriptional modification, and mucosal immunity, and proved that lncRNA can actually encode novel functional micropeptides. However, there are few reports on finding micropeptides encoded by lncRNA in tumors. It is well known that non-coding RNA accounts for up to 90% or more of the total RNA transcribed in the human genome, of which 85% is lncRNA. Therefore, by combining a multi-omics technology and an *in-vivo* and *in-vitro* model cross validation, finding micropeptides encoded by lncRNA in tumors, revealing the specific action and mechanism of the micropeptides in the tumors, and developing a new drug in a targeted manner are of great significance for treating the tumors.

Head and neck tumor is the seventh most malignant tumor worldwide and mainly comprises otolaryngological tumors (laryngeal cancer, nasopharyngeal cancer and paranasal sinus cancer), oral and maxillofacial tumors (tongue cancer, gingival cancer and buccal cancer) and the like. More than 90% of head and neck tumor belong to head and neck squamous cell carcinoma (HNSCC), more than 830 thousand new cases and more than 430 thousand death cases occur each year, and the number of new cases in China is about 1/5 of the total. At present, head and neck cancer is still commonly treated in clinical practice by a combination of operation with radiotherapy and chemotherapy, but the treatment effect is not satisfactory. A targeted treatment drug is mainly an EGFR (epidermal growth factor receptor) inhibitor. Due to high probability of development of drug resistance at a later stage, the clinical efficacy is severely hindered, and the 5-year survival rate of patients with the head and neck cancer is only 50% Therefore, the discovery of new biomarkers and targets for drug development is of important significance for improving the treatment effect of head and neck squamous cell carcinoma.

Renal cell carcinoma (RCC) is a malignant tumor derived from the urinary tubular epithelium of renal parenchyma, accounting for 2%-3% of adult malignant tumors, and the most common type of renal cell carcinoma is clear cell carcinoma, secondly papillary renal cell carcinoma and chromophobe cell carcinoma, and the rare type is such as collecting duct carcinoma. The incidence rate of renal carcinoma, one of the three major tumors of the urinary system, is preceded only by those of prostatic cancer and bladder cancer, but the death rate thereof ranks first among the three major tumors. The incidence of renal carcinoma globally is currently ranked 9th (214 thousand new patients) among all male malignant tumors and 14th (124 thousand new patients) among all female malignant tumors. The latest cancer data released by the National Cancer Center in February 2018 shows that the incidence of renal carcinoma in China is 4.99/100 thousand, wherein the incidence of renal carcinoma in males is 6.09/100 thousand and the incidence of renal carcinoma in females is 3.84/100 thousand. Since the position of kidneys is hidden, the early clinical symptoms are not obvious, and there lacks a recognized diagnostic marker, more than 30% of patients with renal carcinoma have developed to the late stage at initial diagnosis and miss the optimal opportunity for operative radical treatment, such that the search for the specific diagnostic marker of renal carcinoma is an inevitable requirement for improving the early clinical diagnosis rate and carrying out timely treatment.

Prostate carcinoma refers to epithelial malignant tumors occurring in the prostate, including adenocarcinoma, ductal adenocarcinoma, urothelial carcinoma, squamous cell carcinoma, and adenosquamous carcinoma. The prostate adenocarcinoma accounts for more than 95%, and has no symptoms at early stage, and if it has symptoms, it is usually indicative of local invasion or distant metastasis, with focus spread to urethra, bladder and trigone, leading to dysuria. Besides, lumbago, hematuria, emaciation, and weakness may occur during distant metastasis. In recent years, there has been a sharp increase in the incidence of prostate carcinoma. In 2020, prostate carcinoma was only second to lung cancer and the incidence thereof ranked 2nd among men malignant tumors. The clinical diagnosis of prostate carcinoma mainly depends on a digital rectal exam, serum PSA (prostate specific antigen), transrectal ultrasound of the prostate, and pelvic MRI examination, and pathological examination is required to be performed through prostate puncture biopsy before confirmed diagnosis. Patients at early and middle stages are mainly treated by operation and radiotherapy, and patients at a late stage are mainly subjected to endocrinotherapy. Compared with patients in western developed countries, the possibility that patients have developed to the late stage at initial diagnosis is higher in China and patients with prostate carcinoma at the late stage have a poorer prognosis, which pose difficulties for treatment. Therefore, find new diagnostic markers and therapeutic drugs for prostate carcinoma is of great importance for a good prognosis.

Colorectal carcinoma refers to a malignant tumor occurring in mucosal epithelium of colon or rectum, including colon cancer and rectal cancer which are combined together and referred to as colorectal carcinoma in medicine due to similar pathogenesis and diagnosis principle. From the current clinical treatment, with the improvement of urbanization, life style and dietary structure are changing, intake of high-calorie, high-fat, and high-protein food such as chicken, duck and fish meat is increased, aging process of population is accelerated, and thus the problem of high incidence of colorectal tumor becomes more and more prominent. The incidence of colorectal carcinoma in an urban area ranks 2nd-3rd, the ratio of young people under 40 years old suffering from colorectal carcinoma accounts for about 20% of the total population suffering from colorectal carcinoma, and it shows an upward trend. China has been one of the countries with a high incidence of colorectal carcinoma. The high incidence and high mortality of colorectal carcinoma are seriously threatening physical and mental health of people with each passing day, such that the situation for the prevention and treatment of colorectal carcinoma is very serious.

## SUMMARY OF THE INVENTION

The present disclosure provides an antibody specifically binding to a human HMMW micropeptide, comprising a heavy chain variable region comprising complementarity-determining regions CDRH1, CDRH2, and CDRH3, and a light chain variable region comprising complementarity-determining regions CDRL1, CDRL2, and CDRL3, wherein:

(a) CDRH1 has an amino acid sequence as set forth in SEQ ID NO: 1, 11, 21, 31, 41, 51, 61 or 71;
(b) CDRH2 has an amino acid sequence as set forth in SEQ ID NO: 2, 12, 22, 32, 42, 52, 62 or 72;
(c) CDRH3 has an amino acid sequence as set forth in SEQ ID NO: 3, 13, 23, 33, 43, 53, 63 or 73;
(d) CDRL1 has an amino acid sequence as set forth in SEQ ID NO: 4, 14, 24, 34, 44, 54, 64 or 74;
(e) CDRL2 has an amino acid sequence as set forth in SEQ ID NO: 5, 15, 25, 35, 45, 55, 65 or 75; and
(f) CDRL3 has an amino acid sequence as set forth in SEQ ID NO: 6, 16, 26, 36, 46, 56, 66 or 76.

The present disclosure further provides a pharmaceutical composition, comprising the antibody of the present disclosure and a pharmaceutically acceptable carrier.
The present disclosure further provides a nucleic acid molecule, comprising a nucleotide sequence encoding the antibody of the present disclosure.
The present disclosure further provides use of the antibody or the pharmaceutical composition or the nucleic acid molecule of the present disclosure in manufacturing a drug for treating colorectal carcinoma.
The present disclosure further provides use of the antibody or the pharmaceutical composition or the nucleic acid molecule of the present disclosure in manufacturing a kit for diagnosing head and neck squamous cell carcinoma, renal carcinoma, prostate carcinoma, and colorectal carcinoma.

## BRIEF DESCRIPTION OF THE DRAWINGS

The following provides a brief description of the accompanying drawings, which are used to describe the exemplary embodiments disclosed herein, rather than to limit these embodiments.

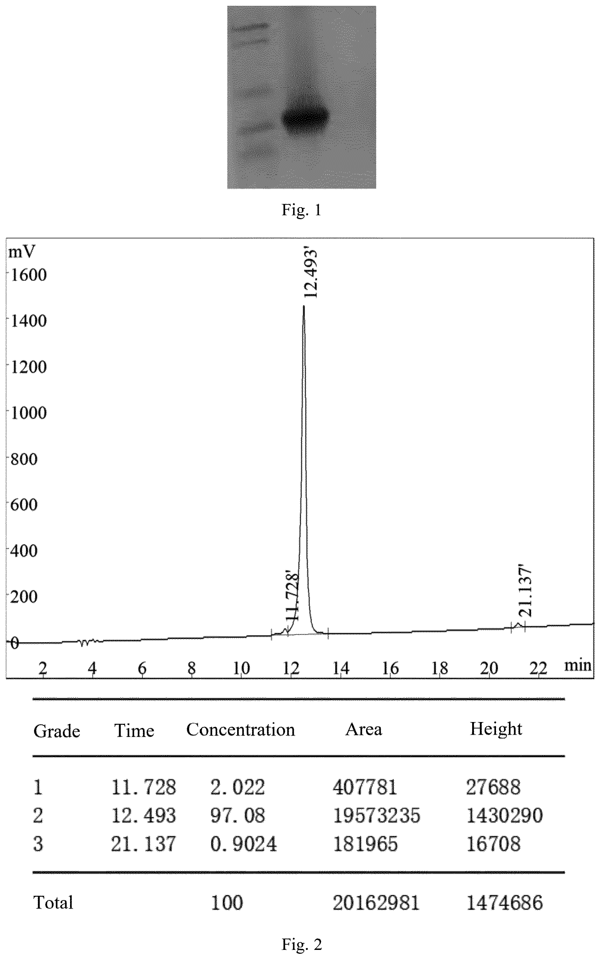

FIG. 1 shows the result of SDS-PAGE electrophoresis of an HMMW micropeptide;
FIG. 2 shows the result of HPLC of the HMMW micropeptide;

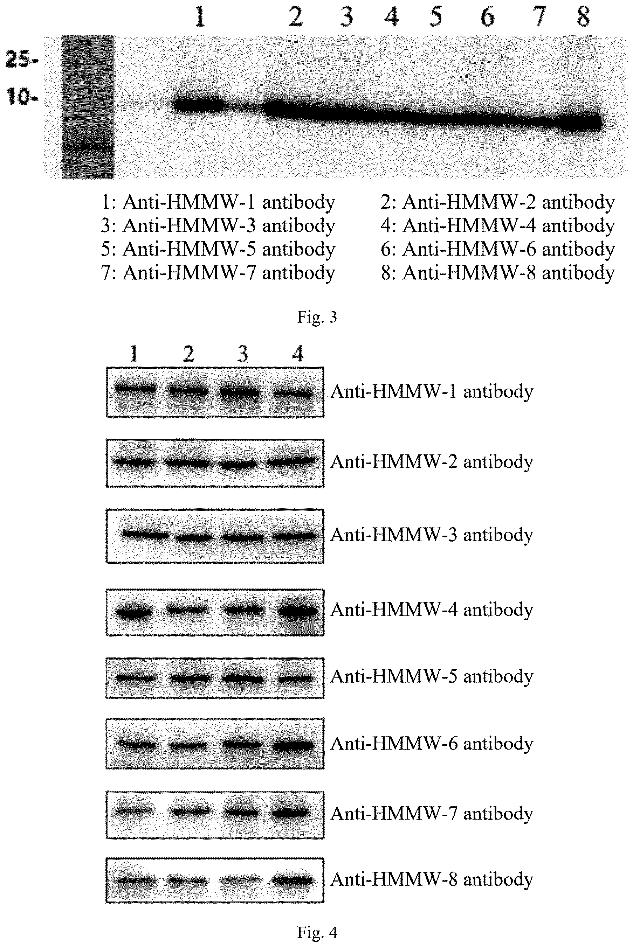

FIG. 3 shows the result of Western Blot of an anti-HMMW-1 antibody, an anti-HMMW-2 antibody, an anti-HMMW-3 antibody, an anti-HMMW-4 antibody, an anti-HMMW-5 antibody, an anti-HMMW-6 antibody, an anti-HMMW-7 antibody, and an anti-HMMW-8 antibody with the fixed amount of the HMMW micropeptide;

FIG. 4 shows the result of Western Blot of the anti-HMMW-1 antibody, the anti-HMMW-2 antibody, the anti-HMMW-3 antibody, the anti-HMMW-4 antibody, the anti-HMMW-5 antibody, the anti-HMMW-6 antibody, the anti-HMMW-7 antibody, and the anti-HMMW-8 antibody with the HMMW micropeptide expressed in CAL27 cells of head and neck squamous cell carcinoma, 786-O cells of renal carcinoma, DU145 cells of prostate carcinoma, and HCT116 cells of colorectal carcinoma;

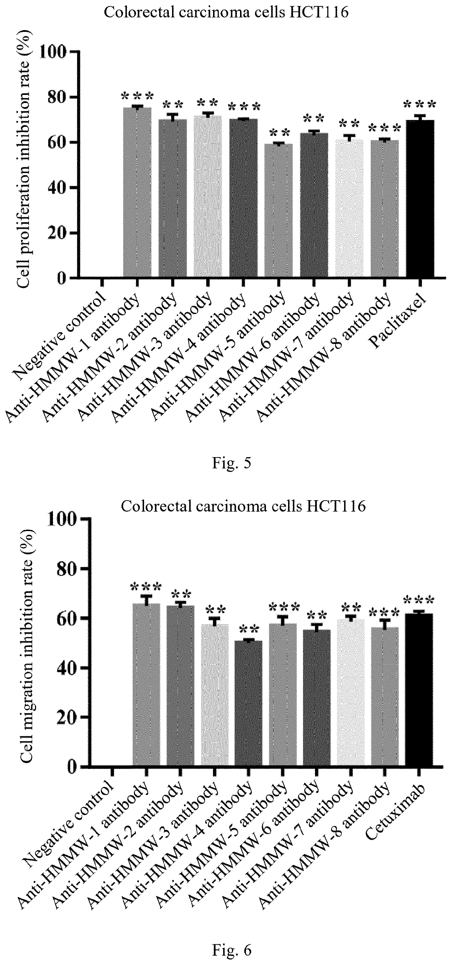

FIG. 5 shows inhibitory effects of the anti-HMMW-1 antibody, the anti-HMMW-2 antibody, the anti-HMMW-3 antibody, the anti-HMMW-4 antibody, the anti-HMMW-5 antibody, the anti-HMMW-6 antibody, the anti-HMMW-7 antibody, and the anti-HMMW-8 antibody on the proliferation of the HCT116 cells of colorectal carcinoma; and

FIG. 6 shows inhibitory effects of the anti-HMMW-1 antibody, the anti-HMMW-2 antibody, the anti-HMMW-3 antibody, the anti-HMMW-4 antibody, the anti-HMMW-5 antibody, the anti-HMMW-6 antibody, the anti-HMMW-7 antibody, and the anti-HMMW-8 antibody on the migration of the HCT 116 cells of colorectal carcinoma.

## DETAILED DESCRIPTION OF EMBODIMENTS

Unless defined otherwise, all technical and scientific terms used herein have the same meanings as commonly understood by one of ordinary skill in the field to which the present disclosure belongs.

When used herein, the expression "A and/or B" includes three cases: (1) A; (2) B; and (3) A and B.

The term "identity" refers to the degree of similarity between a pair of sequences (nucleotides or amino acids). The identity is determined by dividing the number of the same residues by the total number of the residues and multiplying the quotient by 100 to obtain a percentage. Gaps are excluded when assessing identity. Therefore, two copies of completely identical sequences have 100% identity, but sequences with deletion, addition or replacement may have a lower degree of identity. A person skilled in the art will recognize that there are several computer programs that can be used to determine the identity of sequences, such as those programs using algorithms such as BLAST. BLAST nucleotide search is performed using the NBLAST program, and BLAST protein search is performed using the BLASTP program, and default parameters of each program are used.

Two different sequences can be different from each other without affecting the overall function of the protein encoded by the sequence. In this regard, it is well known in the art that chemically similar amino acids can be substituted with each other, usually without changing their functions. Relevant properties can include acidity/alkalinity, polarity/non-polarity, electric charge, hydrophobicity, and chemical structure. For example, alkaline residues Lys and Arg are considered chemically similar and often replace each other. Other examples are acidic residues Asp and Glu, hydroxyl residues Ser and Thr, aromatic residues Tyr, Phe, and Trp, and non-polar residues Ala, Val, Ile, Leu, and Met. These replacements are considered "conservative". Similarly, nucleotide codons and acceptable changes are also known in the art. For example, codons ACT, ACC, ACA, and ACG all encode an amino acid threonine, that is, the third nucleotide can be changed without changing the obtained amino acid. The similarity is determined by dividing the number of the similar residues by the total number of the residues and multiplying the quotient by 100 to obtain a percentage. It should be noted that the similarity and the identity measurements are indicative of different properties.

As used herein, the term "pharmaceutical composition" refers to a mixture of an antibody of the present disclosure and other chemical components, such as a carrier, a stabilizer, a diluent, a dispersant, a suspension, a thickener, and/or an excipient. The pharmaceutical composition facilitates the administration of the antibody to an organism. There are many techniques for administering antibodies in the art, including but not limited to: intravenous, oral, aerosol, parenteral, eye, lung and topical administration.

The term "pharmaceutically acceptable carrier" includes a pharmaceutically acceptable salt, and a pharmaceutically acceptable material, composition or carrier, such as a liquid or solid filler, diluent, excipient, solvent or encapsulating material, and involves carrying or transporting the antibody of the present disclosure within a subject or carrying or transporting the antibody of the present disclosure to a subject so as to perform its intended functions. Each salt or carrier must be "acceptable" regarding compatibility with other components of the preparation and not harmful to the subject. Some examples of materials that can be used as pharmaceutically acceptable carriers include: sugar, such as lactose, glucose, and sucrose; starch, such as corn starch and potato starch; cellulose and derivatives thereof, such as sodium carboxymethyl cellulose, ethyl cellulose, and cellulose acetate; powdered tragacanth gum; malt; gelatin; talc; an excipient, such as cocoa butter and suppository waxes; oil, such as peanut oil, cottonseed oil, safflower oil, sesame oil, olive oil, corn oil and soybean oil; diols, such as propylene glycol; polyols, such as glycerol, sorbitol, mannitol, and polyethylene glycol; esters, such as ethyl oleate and ethyl laurate; agar; a buffering agent, such as magnesium hydroxide and aluminum hydroxide; alginic acid; pyrogen-free water; isotonic saline; a Ringer's solution; ethanol; a phosphate buffer solution; a diluent; a granulating agent; a lubricant; a binder; a disintegrant; a wetting agent; an emulgator; a colorant; a mold release agent; a coating agent; a sweetener; a flavoring agent; an aromatizing agent; a preservative; an antioxidant; a plasticizer; a gelatinizer; a thickener; a hardener; a setting agent; a suspending agent; a surfactant; a humectant; a carrier; a stabilizer; and other non-toxic compatible substances used in pharmaceutical preparations, or any combination thereof.

The term "antibody" refers to a protein used to recognize target antigens by the immune system. The basic functional unit of the antibody is an immunoglobulin monomer. The monomer consists of two identical heavy chains and two identical light chains that form a Y-shaped protein. Each light chain consists of one constant domain and one variable domain. In the light chain, the constant domain can also be called a "constant region", and the variable domain can also be called a "variable region". Each heavy chain consists of one variable domain and three or four constant domains. In the heavy chain, the constant domains together are called "constant regions", while the variable domain can also be called a "variable region". The arms of Y are called fragments, antigen binding (Fab) regions, and each arm is called a Fab fragment. Each Fab fragment consists of one constant domain and one variable domain from the heavy chain, and one constant domain and one variable domain from the light chain. The base of Y is called a Fc region, which consists of two or three constant domains from each heavy chain. The variable domains of the heavy chain and the light chain in the Fab regions are parts for binding antigens of the antibody (such as HMMW in the present disclosure). More specifically, complementarity-determining regions (CDRs) of the variable domains bind their antigens (such as HMMW). In the amino acid sequence of each variable domain, there are three discontinuous CDRs. The term "complete" used herein refers to an antibody containing Fab and Fc regions.

The "antibody heavy chain" used herein refers to the larger of two types of polypeptide chains that exist in the antibody molecule in its natural conformation and usually determines the type of the antibody.

The "antibody light chain" used herein refers to the smaller of two types of polypeptide chains that exist in the antibody molecule in its natural conformation, κ and λ light chains refer to two major isotypes of the antibody light chain.

At present, researches on uses of micropeptides in detection and treatment of head and neck cancer, renal carcinoma, prostate carcinoma, and colorectal carcinoma have not been done. The inventor establishes a micropeptide discovery technical platform by means of transcriptome sequencing data analysis, proteomics, CRISPR/cas9 gene editing, *in-vivo* translation, etc., and discovers the micropeptide HMMW encoded by lncRNA. By screening and analyzing a tumor spectrum with HMMW expression differences, it is found that the expression level of HMMW in head and neck squamous cell carcinoma, renal carcinoma and prostate carcinoma tissues is significantly lower than that in a paracarcinoma tissue, while the expression level of the HMMW in a colorectal carcinoma tissue is significantly higher than that in a paracarcinoma tissue. On this basis, a monoclonal antibody specifically binding to an HMMW micropeptide is developed, can be used for detecting head and neck squamous cell carcinoma, renal carcinoma, prostate carcinoma, and colorectal carcinoma, and provides a new solution for treating colorectal carcinoma. The anti-HMMW antibody of the present disclosure can specifically bind to HMMW in different tumor cells (including but not limited to head and neck squamous cell carcinoma, renal carcinoma, prostate carcinoma and colorectal carcinoma cells), sensitively detects the expression level of an HMMW micropeptide in cells, and thus is effectively used for diagnosis of various cancers. In addition, the anti-HMMW antibody of the present disclosure not only significantly inhibits proliferation of colorectal carcinoma cells, but also significantly inhibits migration of the colorectal carcinoma cells.

In some embodiments, the present disclosure relates to an antibody specifically binding to a human HMMW micropeptide, wherein the antibody comprises a heavy chain variable region comprising complementarity-determining regions CDRH1, CDRH2, and CDRH3, and a light chain variable region comprising complementarity-determining regions CDRL1, CDRL2, and CDRL3, wherein:

(a) CDRH1 has an amino acid sequence as set forth in SEQ ID NO: 1, 11, 21, 31, 41, 51, 61 or 71;
(b) CDRH2 has an amino acid sequence as set forth in SEQ ID NO: 2, 12, 22, 32, 42, 52, 62 or 72;
(c) CDRH3 has an amino acid sequence as set forth in SEQ ID NO: 3, 13, 23, 33, 43, 53, 63 or 73;
(d) CDRL1 has an amino acid sequence as set forth in SEQ ID NO: 4, 14, 24, 34, 44, 54, 64 or 74;
(e) CDRL2 has an amino acid sequence as set forth in SEQ ID NO: 5, 15, 25, 35, 45, 55, 65 or 75; and
(f) CDRL3 has an amino acid sequence as set forth in SEQ ID NO: 6, 16, 26, 36, 46, 56, 66 or 76.

In some embodiments, the present disclosure relates to an antibody specifically binding to a human HMMW micropeptide, wherein the antibody comprises a heavy chain variable region comprising complementarity-determining regions CDRH1, CDRH2, and CDRH3, and a light chain variable region comprising complementarity-determining regions CDRL1, CDRL2, and CDRL3, wherein:

(1) CDRH1 has an amino acid sequence as set forth in SEQ ID NO: 1, CDRH2 has an amino acid sequence as set forth in SEQ ID NO: 2, CDRH3 has an amino acid sequence as set forth in SEQ ID NO: 3, CDRL1 has an amino acid sequence as set forth in SEQ ID NO: 4, CDRL2 has an amino acid sequence as set forth in SEQ ID NO: 5, and CDRL3 has an amino acid sequence as set forth in SEQ ID NO: 6; or

(2) CDRH1 has an amino acid sequence as set forth in SEQ ID NO: 11, CDRH2 has an amino acid sequence as set forth in SEQ ID NO: 12, CDRH3 has an amino acid sequence as set forth in SEQ ID NO: 13, CDRL1 has an amino acid sequence as set forth in SEQ ID NO: 14, CDRL2 has an amino acid sequence as set forth in SEQ ID NO: 15, and CDRL3 has an amino acid sequence as set forth in SEQ ID NO: 16; or

(3) CDRH1 has an amino acid sequence as set forth in SEQ ID NO: 21, CDRH2 has an amino acid sequence as set forth in SEQ ID NO: 22, CDRH3 has an amino acid sequence as set forth in SEQ ID NO: 23, CDRL1 has an amino acid sequence as set forth in SEQ ID NO: 24, CDRL2 has an amino acid sequence as set forth in SEQ ID NO: 25, and CDRL3 has an amino acid sequence as set forth in SEQ ID NO: 26; or

(4) CDRH1 has an amino acid sequence as set forth in SEQ ID NO: 31, CDRH2 has an amino acid sequence as set forth in SEQ ID NO: 32, CDRH3 has an amino acid sequence as set forth in SEQ ID NO: 33, CDRL1 has an amino acid sequence as set forth in SEQ ID NO: 34, CDRL2 has an amino acid sequence as set forth in SEQ ID NO: 35, and CDRL3 has an amino acid sequence as set forth in SEQ ID NO: 36; or

(5) CDRH1 has an amino acid sequence as set forth in SEQ ID NO: 41, CDRH2 has an amino acid sequence as set forth in SEQ ID NO: 42, CDRH3 has an amino acid sequence as set forth in SEQ ID NO: 43, CDRL1 has an amino acid sequence as set forth in SEQ ID NO: 44, CDRL2 has an amino acid sequence as set forth in SEQ ID NO: 45, and CDRL3 has an amino acid sequence as set forth in SEQ ID NO: 46; or

(6) CDRH1 has an amino acid sequence as set forth in SEQ ID NO: 51, CDRH2 has an amino acid sequence as set forth in SEQ ID NO: 52, CDRH3 has an amino acid sequence as set forth in SEQ ID NO: 53, CDRL1 has an amino acid sequence as set forth in SEQ ID NO: 54, CDRL2 has an amino acid sequence as set forth in SEQ ID NO: 55, and CDRL3 has an amino acid sequence as set forth in SEQ ID NO: 56; or

(7) CDRH1 has an amino acid sequence as set forth in SEQ ID NO: 61, CDRH2 has an amino acid sequence as set forth in SEQ ID NO: 62, CDRH3 has an amino acid sequence as set forth in SEQ ID NO: 63, CDRL1 has an amino acid sequence as set forth in SEQ ID NO: 64, CDRL2 has an amino acid sequence as set forth in SEQ ID NO: 65, and CDRL3 has an amino acid sequence as set forth in SEQ ID NO: 66; or

(8) CDRH1 has an amino acid sequence as set forth in SEQ ID NO: 71, CDRH2 has an amino acid sequence as set forth in SEQ ID NO: 72, CDRH3 has an amino acid sequence as set forth in SEQ ID NO: 73, CDRL1 has an amino acid sequence as set forth in SEQ ID NO: 74, CDRL2 has an amino acid sequence as set forth in SEQ ID NO: 75, and CDRL3 has an amino acid sequence as set forth in SEQ ID NO: 76.

In some embodiments, the antibody heavy chain variable region comprises an amino acid sequence as set forth in SEQ ID NO: 7, 17, 27, 37, 47, 57, 67 or 77 or an amino acid sequence having at least 90%, at least 95%, at least 96%, at least 97%, at least 98%, at least 99% or at least 99.5% sequence identity with the amino acid sequence as set forth in SEQ ID NO: 7, 17, 27, 37, 47, 57, 67 or 77. In some embodiments, the amino acid sequence of the antibody heavy chain variable region is set forth in SEQ ID NO: 7, 17, 27, 37, 47, 57, 67 or 77.

In some embodiments, the antibody light chain variable region comprises an amino acid sequence as set forth in SEQ ID NO: 8, 18, 28, 38, 48, 58, 68 or 78 or an amino acid sequence having at least 90%, at least 95%, at least 96%, at least 97%, at least 98%, at least 99% or at least 99.5% sequence identity with the amino acid sequence as set forth in SEQ ID NO: 8, 18, 28, 38, 48, 58, 68 or 78. In some embodiment, the amino acid sequence of the antibody light chain variable region is set forth in SEQ ID NO: 8, 18, 28, 38, 48, 58, 68 or 78.

In some embodiments, the antibody heavy chain variable region comprises an amino acid sequence as set forth in SEQ ID NO: 7, 17, 27, 37, 47, 57, 67 or 77 and the antibody light chain variable region comprises an amino acid sequence as set forth in SEQ ID NO: 8, 18, 28, 38, 48, 58, 68 or 78. In some embodiments, the antibody heavy chain variable region comprises an amino acid sequence as set forth in SEQ ID NO: 7 and the antibody light chain variable region comprises an amino acid sequence as set forth in SEQ ID NO: 8. In some embodiments, the antibody heavy chain variable region comprises an amino acid sequence as set forth in SEQ ID NO: 17 and the antibody light chain variable region comprises an amino acid sequence as set forth in SEQ ID NO: 18. In some embodiments, the antibody heavy chain variable region comprises an amino acid sequence as set forth in SEQ ID NO: 27 and the antibody light chain variable region comprises an amino acid sequence as set forth in SEQ ID NO: 28. In some embodiments, the antibody heavy chain variable region comprises an amino acid sequence as set forth in SEQ ID NO: 37 and the antibody light chain variable region comprises an amino acid sequence as set forth in SEQ ID NO: 38. In some embodiments, the antibody heavy chain variable region comprises an amino acid sequence as set forth in SEQ ID NO: 47 and the antibody light chain variable region comprises an amino acid sequence as set forth in SEQ ID NO: 48. In some embodiments, the antibody heavy chain variable region comprises an amino acid sequence as set forth in SEQ ID NO: 57 and the antibody light chain variable region comprises an amino acid sequence as set forth in SEQ ID NO: 58. In some embodiments, the antibody heavy chain variable region comprises an amino acid sequence as set forth in SEQ ID NO: 67 and the antibody light chain variable region comprises an amino acid sequence as set forth in SEQ ID NO: 68. In some embodiments, the antibody heavy chain variable region comprises an amino acid sequence as set forth in SEQ ID NO: 77 and the antibody light chain variable region comprises an amino acid sequence as set forth in SEQ ID NO: 78.

In some embodiments, the antibody light chain variable region and/or heavy chain variable region is/are a part of a single-chain variable fragment (scFv), a F(ab')$_2$ fragment, a Fab or Fab' fragment, a bivalent antibody, a trivalent antibody, a tetravalent antibody or a monoclonal antibody.

The scFv includes a light chain variable region and a heavy chain variable region that are usually joined together by a linker. The linker is usually about 10 amino acids to about 25 amino acids in length (although it does not have to be in this range). An N-terminal of one variable domain is connected to a C-terminal of other variable domains. It is desirable that the scFv is subjected to PEGylation (i.e., treated with polyethylene glycol) to increase its size, as certolizumab pegol. Two scFvs can be bonded together by another linker to produce a tandem scFv.

If the light chain variable region and the heavy chain variable region are joined together by a short linker to form scFv, the two variable regions cannot be overlapped, otherwise the scFv will undergo dimerization to form a bivalent antibody. Even shorter linkers can lead to the formation of trimers (i.e., a trivalent antibody) and tetramers (i.e., a tetravalent antibody).

A complete monoclonal antibody consists of two heavy chains and two light chains. Besides, each light chain and each heavy chain contain variable domains. Each light chain is joined with a heavy chain. Two heavy chains are joined together in a hinge region. If a heavy chain constant region below the hinge region is removed, a $F(ab')_2$ fragment containing a total of four variable regions is generated. The $F(ab')_2$ fragment can be divided into two Fab' fragments. The Fab' fragments contain sulfhydryl groups from the hinge region. When the heavy chain constant region above the hinge region is removed, a Fab fragment is formed and does not contain sulfhydryl groups from the hinge region. However, all these fragments contain light chain variable region and heavy chain variable region.

In some embodiments, the antibody of the present disclosure is a complete monoclonal antibody formed by combining the above-mentioned light chains and heavy chains containing variable regions/domains with human constant regions. The heavy chain constant region can be any human isotypes, including IgAl, IgA2, IgD, IgE, IgG1, IgG2, IgG3, IgG4 or IgM. The human light chain constant region can be κ or λ isotypes.

The above-mentioned various embodiments and preferences for the antibody in the present disclosure can be combined with each other (as long as they are not inherently contradictory to each other), and the various embodiments formed by the combination are considered as a part of the present disclosure.

In some embodiments, the present disclosure relates to a pharmaceutical composition, comprising the antibody of the present disclosure and a pharmaceutically acceptable carrier.

In some embodiments, the pharmaceutical composition comprises a pharmaceutically acceptable carrier. The carrier acts as a medium for delivering an antibody. Examples of the pharmaceutically acceptable carrier include a liquid carrier (such as water, oil, and alcohols) in which an antibody can be dissolved or suspended.

The pharmaceutical composition may also include an excipient. Specifically, the excipient includes a buffer, a surfactant, a preservative, a filler, a polymer, and a stabilizer, which can be used together with the antibody. The buffer is used to control the pH value of the composition. The surfactant is used to stabilize proteins, inhibit protein aggregation, inhibit protein adsorption to the surface, and assist in protein refolding. Examples of the surfactant include Tween 80, Tween 20, Brij 35, Triton X-10, PluronicF127, and sodium dodecyl sulfate. The preservative is used to inhibit microbial growth. Examples of the preservative include benzyl alcohol, m-cresol, and phenol. The filler is used during freeze-drying to increase volume. The hydrophilic polymer (such as dextran, hydroxyethyl starch, polyethylene glycol, and gelatin) can be used to stabilize proteins. The polymer with nonpolar parts (such as polyethylene glycol polymers) can also be used as a surfactant. The protein stabilizer may include polyols, sugars, amino acids, amines, and salts. The suitable sugars include sucrose and trehalose. The amino acids include histidine, arginine, glycine, methionine, proline, lysine, glutamate, and a mixture thereof. Proteins such as human serum albumin can also be competitively adsorbed on the surface and reduce antibody aggregation. It should be noted that specific molecules can be used for many purposes. For example, histidine can be used as a buffer and an antioxidant. Glycine can be used as a buffer and a filler.

The antibody or composition disclosed herein can be properly developed to be suitable for the following administration routes, including inhalation, oral, nasal, rectal, parenteral, sublingual, percutaneous, permucosal (e.g., sublingual, lingual, buccal/perbuccal, urethral/transurethral, vaginal (e.g., transvaginal and endovaginal), nasal/transnasal and rectal/transrectal), intravesical, intrapulmonary, transduodenal, intragastric, intrathecal, subcutaneous, intramuscular, intradermal, intraarterial, intravenous, intrabronchial, inhalation, and topical administration. Suitable compositions and dosage forms include, for example, a tablet, a capsule, a cachet, a pill, a gel cap, a buccal tablet, a dispersion, a suspension, a solution, a syrup, a granule, a bead, a transdermal patch, a gel, a powder, a pellet, a slurry, a pastille, a cream, a paste, an ointment, a lotion, a plate, a suppository, a liquid spray for nasal or oral administration, a dry powder or an aerosol preparation for inhalation, and a composition and a preparation for intravesical administration. It should be understood that preparations and compositions can be used in the present disclosure are not limited to the specific preparations and compositions described herein.

In some embodiments, the present disclosure relates to a nucleic acid molecule, comprising a nucleotide sequence encoding the antibody of the present disclosure.

In some embodiments, the present disclosure relates to a nucleic acid molecule, comprising a heavy chain variable region coding sequence as set forth in SEQ ID NO: 9, 19, 29, 39, 49, 59, 69 or 79 or a nucleotide sequence having at least 90%, at least 95%, at least 96%, at least 97%, at least 98%, at least 99% or at least 99.5% sequence identity with the heavy chain variable region coding sequence as set forth in SEQ ID NO: 9, 19, 29, 39, 49, 59, 69 or 79, and/or a light chain variable region coding sequence as set forth in SEQ ID NO: 10, 20, 30, 40, 50, 60, 70 or 80 or a nucleotide sequence having at least 90%, at least 95%, at least 96%, at least 97%, at least 98%, at least 99% or at least 99.5% sequence identity with the light chain variable region coding sequence as set forth in SEQ ID NO: 10, 20, 30, 40, 50, 60, 70 or 80.

In some embodiments, the present disclosure relates to a nucleic acid molecule, comprising a heavy chain variable region coding sequence as set forth in SEQ ID NO: 9, 19, 29, 39, 49, 59, 69 or 79 and/or a light chain variable region coding sequence as set forth in SEQ ID NO: 10, 20, 30, 40, 50, 60, 70 or 80.

In some embodiments, the present disclosure relates to use of the antibody or the pharmaceutical composition or the nucleic acid molecule of the present disclosure in manufacturing a drug for treating colorectal carcinoma.

In some embodiments, the present disclosure relates to use of the antibody or the pharmaceutical composition or the nucleic acid molecule of the present disclosure in manufacturing a kit for diagnosing head and neck squamous cell carcinoma, renal carcinoma, prostate carcinoma, and colorectal carcinoma.

The various embodiments and preferences for the antibody in the present disclosure are also applicable to the composition, the nucleic acid molecule, and the use of the present disclosure, these embodiments and preferences can also be combined with each other (as long as they are not inherently contradictory to each other), and the various embodiments formed by the combination are considered as a part of the present application.

The technical solution of the present disclosure will be more clearly and explicitly described by way of illustration in combination with examples. It should be understood that these examples are only for illustrative purposes and not intended to limit the protection scope of the present disclosure. The protection scope of the present disclosure is only defined by the appended claims.

## EXAMPLES

### Example 1: Construction and expression of recombinant vector for HMMW micropeptide

A nucleotide sequence (SEQ ID NO. 82) corresponding to an HMMW micropeptide (the amino acid sequence is set forth in SEQ ID NO. 81) was synthesized by General Biological System (Anhui) Co., Ltd. (hereinafter referred to as General Biological). Through EcoRI--BamHI double enzyme digestion (NEB, USA), the synthesized nucleotide sequence was linked to an expression vector pET28a (Addgene, USA), the linked product was transformed into *Escherichia coli* DH5α which was then applied on a plate, several clones were picked, the bacteria were subjected to a shaking culture, plasmids were extracted with a Beijing Tiangen biological plasmid extraction kit, and a recombinant expression vector with a correct expression clone sequence was confirmed by sequencing of General Biological. The obtained expression plasmids were transformed into *Escherichia coli* BL21 (Beijing TransGen Biotech Co., Ltd.). The transformation included the following specific steps: 1) 1 μL of the plasmids were added into competent cells, mixed evenly, and the mixture was placed on ice for 30 min; 2) the mixture was subjected to water bath at 42°C for 90 s, taken out immediately, and placed on ice for 2 min; 3) the mixture was all inoculated into 1 mL of an LB medium; 4) the medium was cultured at 37°C for 45 min in a shaker at 180 rpm to recover the resistance; and 5) the culture was centrifuged at 5,000 rpm × 5 min. A supernatant was discarded, 100 μL was left and mixed evenly, the mixture was applied on a plate (LB-Amp+), the plate was firstly placed upwardly and absorbed for 20 min, then the plate was inverted for 12 h, and a single colony was picked and inoculated to 5 mL of an LB (×2) medium, subjected to a shaking culture at 37°C. When the absorbance value OD600 of the bacterial solution was about 0.6, IPTG (Sigma-Aldrich) was added for a further induction culture for 3 h. The bacterial solution was centrifuged at 5,000 rpm for 5 min, the bacteria were collected and washed with PBS, the bacteria were centrifuged and collected, and the bacteria were disintegrated by ultrasonic treatment at 4°C. The disintegrated bacteria were centrifuged at 10,000 rpm, the supernatant was collected for an SDS-PAGE electrophoresis, and the result was shown in FIG. 1, indicating expression of the HMMW micropeptide. The HMMW micropeptide was purified by nickel ion affinity chromatography and imidazole gradient elution, and the HPLC detection result was shown in FIG. 2, indicating that purity of the HMMW micropeptide is higher than 90%.

### Example 2: Preparation of monoclonal antibody of anti-human HMMW micropeptide

8-12 week old BALB/c mice (Changzhou Cavens Experimental Animal Co., Ltd.) were purchased and immunized with the purified HMMW micropeptide with the amount of immunogen of about 50 μg/mouse/time. During the first immunization, the antigen emulsified with a Freund's complete adjuvant (Sigma-Aldrich) was injected subcutaneously at multiple points on the back of the neck. Three weeks after the first immunization, the immunogen emulsified with the same volume of the Freund's incomplete adjuvant (Sigma-Aldrich) was used for the second and third immunization about every other 2 weeks with the same immunization dose as that of the first immunization. After three times of immunization, blood was collected from the venous plexus of mouse eyes, an ELISA titer (protein antigen-coated plate) of antiserum was detected, and the mice with the antiserum titer greater than 8 K were selected for a fusion stage.

One day before the fusion, one 8-12 week old BALB/c mouse was taken and placed in a 75% ethanol solution for 2 min. The spleen of the mouse was taken out aseptically and placed in a 200-mesh stainless steel screen. A single cell suspension was obtained by grinding. A single cell suspension of the immunized mice was obtained by the same method. SP2/0 cells that grew well and were at exponential phase were collected and mixed with spleen cells at a ratio of 1 : 5 in a 50-ml transparent plastic centrifuge tube, the mixture was washed with a preheated RPMI 1640 basic culture medium (Biological Industries, Israel) once (1,400 rpm, 5 min), the supernatant was discarded, and the bottom of the tube was gently tapped with a finger to fully mix the two cells as a cell suspension. The centrifuge tube was placed in a 37°C heat-preservation water bath cup for preheating, 1 ml of a 50% PEG solution preheated at 37°C was absorbed and added at a constant rate within 1 min, and the centrifuge tube was gently shaken while adding and then gently shaken in a 37°C water bath for 60 s. 14 ml of a 1640 basic culture medium preheated at 37°C was gently and evenly dropwise added along the tube wall to terminate the reaction (1 ml was added within the first minute and 3 ml within 3 min, and finally 10 ml was slowly added). After standing at 37°C for 5 min, the mixture was centrifuged (800 rpm, 5 min), the supernatant was discarded (the centrifuge tube was tilted and the supernatant was aspirated), the precipitated cells were gently suspended in a 1640 selective medium preheated at 37°C (no pipetting), after even mixing, the mixture was dropwise added into the 96-well culture plate containing trophoblasts with 100 μl/well, the cells were cultured in a 5% $CO_2$ incubator at 37°C, half amount of the solution was changed after 3 days, and the cells were cultured with an HT medium (Thermo Fisher Scientific, USA) after 10 days and cultured with a 1640 medium containing 10% FBS (Biological Industries, Israel) instead after 2 weeks. During the period, the growth of clones in the 96-well plate was observed every day. When hybridoma cells cover 1/10 area of the well bottom, detection of a specific antibody was started, and the required hybridoma cell line was screened out. The above steps were repeated until the positive rate of the cell line in the well was 100% (recognizing the corresponding antigen), and 8 hybridoma cell lines were obtained.

**Example 3: Sequencing of light chain variable region and heavy chain variable region of anti-human HMMW antibodies**

RNA was extracted from the 8 hybridoma cell lines (an RNA extraction kit was purchased from Baoriyi Biotechnology Co., Ltd.), cDNA was subjected to reverse transcription by using 5'RACE (Baoriyi Biotechnology Co., Ltd.), and a heavy chain variable region (VH) and a light chain variable region (VL) of the hybridoma cells were cloned by PCR, and then sequenced. The sequencing result was shown in Table 1 below, wherein CDRs were shown in the shaded parts.

Table 1

| Name | Region | SEQ ID NO | Sequence |
|---|---|---|---|
| Anti-H MMW antibody -1 | VH | 7 | FLFSDKHRNRTFTMYLGLNCVFIVFLLKGVQSEVKLEESGGGLVQPG GSMKFSCVASGFTFSNYWMNWVRQSPERGLEWVAEIKLKSNNYATL YAESVKGRFTISRDDSKSSVYLQVNNLRAEDTGIYYCITEIFDYWGQG TTLTVSSAKTTPPSVYPLAP |
| | VL | 8 | SLILSLLALSSGAISQAVVTQESALTTSPGETVTLTCRSSTGAVTTSNY ATWVQEKPDHLFTGLIVGANNRAPGVPARFSGSLIGDKAALTITGAQT EDEAIYFCALWFLNHWVFGGGTKLTVLGQPKSSPSV |
| Anti-H MMW antibody -2 | VH | 17 | TTLDSQVFLFSDKHRNRTFTMYLGLNCVFIVFLLKGVQSEVKLEESGG GLVQPGGSMKVSCVGSGFTFSNYWMNWVRQSPEKGLEWVAEIRLKS NNYATHYAESVKGRFTISRDDSKRSVYLQMNNLRVEDTGIYYCTTEA VDYWGQGTSVTVSSAKTTPPSVYPLAP |
| | VL | 18 | DSPYQVRRMRFSAQLLGLLVLWIPGSTADIVMTQAAFSNPVTLGTSAS ISCRSSKSLLHSNGITYLYWYLQKPGQSPQLLIYQMSNLASGVPDRFSS SGSGTDFTLRISRVEAEDVGVYYCAQNLELPTFGGGTKLEIKRADAAP TVS |
| Anti-H MMW antibody -3 | VH | 27 | EVKLEESGGGLVQPGGSMKLSCVASGFTFSDYWMNWVRQSPEKGLE WLTEIRLKSDNYATHYAESVKGKFTISRDDSKSTVYLQMNNLRAEDT GIYYCSVETMDFWGQGTSVTVSS |
| | VL | 28 | QAVVTQESALTTSPGETVTLTCRSSIGAVTTNNYANWVQEKPDRLFT GLIRGTNNRVPGVPARFSGSLIGDKAALTITGAQTEDEAIYFCALWYS NHWVFGGGTKLTVL |
| Anti-H MMW antibody -4 | VH | 37 | EVKLEESGGGLVQPGGSMKLSCVASGFTFSDYWMNWVRQSPEKGLE WLTEIRLKSNNYLTHYAESVKGRFTISRDDSKSSVYLQMNNLRAEDT GIYYCSVETMDYWGQGISVTVSS |
| | VL | 38 | QAVVTQESALTTSSGETVTLTCRSSTGAVTTSNYANWVQEKPDHLFT GLIRGTNNRAPGVPARFSGSLIGDKAALTITGAQTEDEAIYFCALWYS NHWVFGGGTKLTVL |
| Anti-H MMW antibody -5 | VH | 47 | EVKLEESGGGLVQPGGSMKLSCVVSGFTFSNYWMNWVRQSPERGLE WVAEIKLKSNNYATLYAASVKGRFTISRDDSKSSVYLQVNNLRTEDT GIYYCITEIFDYWGQGTTLTVSS |
| | VL | 48 | QTVVTQESALTTSPGETVTLTCRSSTGSVTTSNYATWVQEKPDHLFTG LIVGANNRAPGVPARFSGSLIGDKAALTITGAQTEDEAIYFCALWFIDH WVFGGGTKLTVL |

| Anti-HMMW antibody-6 | VH | 57 | EVKLEESGGGLVQPGGSMKLSCVAS GFTFSNYW MNWVRQSPEKGLE WVAE IRLKSNNYAT HYAESVTGRFTISRDDSKSSVYLQMNNLRAEDT GIYYC TLEAVDY WGQGTSVTVSS |
|  | VL | 58 | DIVMTQAAFSNPVTLGTSASISCRSS KSLLHSDGITY LYWFLQKPGQSP QLLIY QMSNLAS GVPDRFSSSGSGTDFTLRISRVEAEDVGVYY CAQNL ELPTF GGGTKLEIK |
| Anti-HMMW antibody-7 | VH | 67 | EVKLEESGGGLVQPGGSKKLSCFAS GFTFSNYW MNWVRQSPEKGLE WVAE IQLKSNNYAT HYAESVKGRFTISRDDSKSSVYLQMNNLRAEDT GIYYC TTEIFDF WGQGTTLTVSS |
|  | VL | 68 | DVVMTQTPLTLSVTIGQPASISCKSS QSLLHSDGKTY LSWLLQRPGQS PKRLIY LVS KLDSGVPDRFTGSGSGTDFTLKISRVEAEDLGVYYC WQG IHFPHT FGGGTKLEIK |
| Anti-HMMW antibody-8 | VH | 77 | EVKLEESGGGLVQPGGSMKVSCVAS GFTFSNYW MNWVRQSPEKGLE WVAE IKLKSDIDAT NYAESVKGRFTISRDDSKSSVSLQMNNLRAEDT GIYYC STEIFDY WGQGTTLTVSS |
|  | VL | 78 | DVVMTQTPLTLSVTIGQPASISCKSS QSLLHSDGKTY LSWLLQRPGQS PKRLIY LVS KLDSGVPDRFTGSGSGTDFTLKISRVEAEDLGIYYC CQYI HFPHT FGGGTKLEIK |

## Example 4: Detection of anti-HMMW antibody titers by ELISA

The antigen coating concentration of the HMMW micropeptide was 1 μg/ml and 5% milk PBS-T was used for sealing. The dilution multiples of eight antibodies were: 1: 3.125 K/6.25 K/12.5 K/25 K/50 K/100 K. The primary antibodies were incubated at 37°C for 1 h and the secondary antibodies were incubated at 37°C for 45 min. A color developing solution was added, reaction was performed at 37°C for 15 min, the reaction was terminated, and the value was read. It can be seen from Table 2 below that the titers of the eight antibodies all reached 1: 100 K, meeting the requirements for subsequent antigen detection.

Table 2

| Number of antibody | 3.125 K | 6.25 K | 12.5 K | 25 K | 50 K | 100 K | Positive well | Blank well |
|---|---|---|---|---|---|---|---|---|
| Anti-HMMW antibody-1 | 2.85 | 2.59 | 2.38 | 2.13 | 1.85 | 1.14 | 3.30 | 0.0392 |
| Anti-HMMW antibody-2 | 3.33 | 3.35 | 3.23 | 3.19 | 2.94 | 2.73 | 3.67 | 0.0437 |
| Anti-HMMW antibody-3 | 3.57 | 3.40 | 3.22 | 3.04 | 2.92 | 2.54 | 3.78 | 0.0629 |
| Anti-HMMW antibody-4 | 3.52 | 3.45 | 3.35 | 3.13 | 2.81 | 2.57 | 3.82 | 0.0474 |
| Anti-HMMW antibody-5 | 2.92 | 2.81 | 2.56 | 2.48 | 2.31 | 2.00 | 3.20 | 0.0434 |
| Anti-HMMW antibody-6 | 3.37 | 3.19 | 2.96 | 2.55 | 2.20 | 2.16 | 3.39 | 0.0462 |
| Anti-HMMW antibody-7 | 2.40 | 2.26 | 2.27 | 2.15 | 2.14 | 1.70 | 3.10 | 0.0875 |
| Anti-HMMW antibody-8 | 3.45 | 3.16 | 3.11 | 2.97 | 2.96 | 2.79 | 3.45 | 0.0889 |

## Example 5: Improvement of sensitivity in detecting anti-HMMW antibodies by Western Blot

The purified HMMW micropeptide was subjected to a 15% SDS-PAGE gel electrophoresis with a total loading amount of 10 ng, the obtained product was then transferred to an NC membrane, and the membrane was sealed with 5% skimmed milk powder at room temperature for 1 h, incubated with 8 diluted primary antibodies at room temperature for 2 h, washed with TBST four times, incubated with the secondary antibodies at room temperature for 45 min, and washed with TBST three times, and the film was washed. An ECL reagent was added to react for 1 min, the product was sealed with a preservative film, an X-ray film was exposed in a dark room for development and scanned, and the result was shown in FIG. 3. It can be seen from FIG. 3 that all the eight anti-HMMW antibodies can recognize 10 ng of the HMMW micropeptide, and thus the sensitivity meets the detection requirement.

**Example 6: Detection of capability of anti-HMMW antibodies for binding to HMMW micropeptide in different tumor cells by Western Blot**

CAL27 cells of head and neck squamous cell carcinoma, 786-O cells of renal carcinoma, DU145 cells of prostate carcinoma, and HCT116 cells of colorectal carcinoma (all from the American type culture collection) were separately cultured in an 5% $CO_2$ incubator at 37°C to a density of 90%. The cells were digested with trypsin (Biosharp, USA) and collected. The digested cells were centrifuged, the supernatant was discarded, the residue was rinsed with PBS, and the supernatant was discarded. RIPA lysis buffer was added and the mixture was lysed on ice for 20 min. The lysed solution was centrifuged at 12,000 g for 10 min and the supernatant was collected. $1\times$ SDS loading buffer solution was added, the solution was pipetted and mixed evenly, and the mixture was boiled for denaturation for 5 min. Total protein was separated by 10% SDS-PAGE gel, and then transferred to a PVDF membrane (Millipore, USA). The membrane was sealed with 5% BSA at room temperature for 2 h, incubated with 8 different anti-HMMW antibodies at 4°C overnight, and washed with TBST for 3 times. The membrane was incubated with a secondary antibody at room temperature for 1 h and washed with TBST for 3 times. The obtained product was developed with a hypersensitive chemiluminescence (ECL) solution and imaged by a Tanon imaging system. The capability of the different anti-HMMW antibodies for detecting the expression of the HMMW micropeptide in the four tumor cells was compared, and the result was shown in FIG. 4.

It can be seen from FIG. 4 that the eight anti-HMMW antibodies can effectively detect the expressions of the HMMW micropeptide in the CAL27 cells of head and neck squamous cell carcinoma, the 786-O cells of renal carcinoma, the DU145 cells of prostate carcinoma, and the HCT116 cells of colorectal carcinoma.

**Example 7: Effect of anti-HMMW antibodies on proliferation of human colorectal carcinoma cells**

The colorectal carcinoma cells HCT116 with a high expression level of the HMMW micropeptide were cultured in a 5% $CO_2$ incubator at 37°C to a density of 90%, the cells were digested with trypsin and collected. The cells were resuspended in a culture solution and counted under a microscope. The cell concentration was adjusted to $3.0 \times 10^4$ cell/mL and the cell suspension was inoculated into a 96-well plate with 100 $\mu$l per well. The cells were cultured overnight in the 5% $CO_2$ incubator at 37°C. After the cells completely adhered to the wall, 40 nM of one of the eight anti-HMMW antibodies was respectively added as an administration group, 10 $\mu$g/ml of paclitaxel was added as a positive control group, and the culture solution without addition of any drug was as a negative control group, with 100 $\mu$l per well, cultured in the 5% $CO_2$ incubator at 37°C for 48 h. 20 $\mu$L of 5 mg/mL of MTT was added into each well of a 96-well plate and the cells were continuously cultured for 4 h. The culture medium was aspirated and the cells were dissolved with 100 $\mu$L of DMSO per well. The absorbance value at a detection wavelength of 570 nm and a reference wavelength of 630 nm was measured using a microplate reader. Proliferation inhibition (PI) was calculated using the formula of PI(%) = 1-(Administration group or Positive control group)/Negative group. The experiment was repeated 3 times independently. The obtained result by the experiment was expressed by mean $\pm$ standard deviation and subjected to a statistical T test. *P < 0.05 was considered a significant difference and **P < 0.01 was considered an extremely significant difference, as shown in FIG. 5.

It can be clearly seen from FIG. 5 that compared with the negative control, the eight anti-HMMW antibodies at a dose of 40 nM can all significantly inhibit the proliferation of the colorectal carcinoma cells HCT116, with the inhibitory effect basically equivalent to that of paclitaxel, an anticancer drug widely used in clinical treatment of cancer, and thus are proved to be used as an effective anticancer drug.

**Example 8: Effect of anti-HMMW antibodies on migration of human colorectal carcinoma cells**

The colorectal carcinoma cells HCT116 with a high expression level of the HMMW micropeptide were inoculated into a transwell chamber (Millipore, USA) with 100 $\mu$L per well, at the same time, 100 $\mu$L of 40 nM of one of the eight anti-HMMW antibodies was respectively added into each chamber as an administration group, 40 nM of cetuximab was added as a positive control group, and the culture solution without addition of any drug was as a negative control group. Then 0.6 mL of a complete culture medium containing 10% FBS was added into the transwell chamber to stimulate cell migration, and the cells were cultured at 5% $CO_2$ and 37°C for 24 h. The culture solution in the wells was discarded. The cells were fixed with 90% alcohol at room temperature for 30 min, dyed with 0.1% of crystal violet at room temperature for 10 min, and rinsed clean with water. The upper layer of non-migrated cells were gently wiped off with a cotton swab. The cells were observed under a microscope, four visual fields were selected to be photographed, and the cells were counted. Migration inhibition rate (MIR) of cells was calculated according to the formula:

$$MIR(\%) = 1 - \cfrac{N_{test}}{N_{negative\ control}} \times 100\%$$

wherein, $N_{test}$ is the migration number of cells in a test group (the antibody administration groups or the positive control group), and $N_{control}$ is the migration number of cells in the negative control group. The result was shown in FIG. 6.

It can be clearly seen from FIG. 6 that compared with the negative control, the eight anti-HMMW antibodies at a dose of 40 nM can all significantly inhibit the migration of the colorectal carcinoma cells HCT116, with the inhibitory effect basically equivalent to that of cetuximab, an anticancer drug widely used in clinical treatment of cancer, and thus are proved to be a therapeutic drug capable of effectively inhibiting migration of malignant tumor cells.

Although the specific embodiments have been described, for the applicant or a person skilled in the art, the substitutions, modifications, changes, improvements, and substantial equivalents of the above embodiments may exist or cannot be foreseen currently. Therefore, the submitted appended claims and claims that may be modified are intended to cover all such substitutions, modifications, changes, improvements, and substantial equivalents.

**Claims**

1. An antibody specifically binding to a human HMMW micropeptide, wherein the antibody comprises a heavy chain variable region comprising complementarity-determining regions CDRH1, CDRH2, and CDRH3, and a light chain variable region comprising complementarity-determining regions CDRL1, CDRL2, and CDRL3, wherein:

   (a) the CDRH1 has an amino acid sequence as set forth in SEQ ID NO: 1, 11, 21, 31, 41, 51, 61 or 71;
   (b) the CDRH2 has an amino acid sequence as set forth in SEQ ID NO: 2, 12, 22, 32, 42, 52, 62 or 72;
   (c) the CDRH3 has an amino acid sequence as set forth in SEQ ID NO: 3, 13, 23, 33, 43, 53, 63 or 73;
   (d) the CDRL1 has an amino acid sequence as set forth in SEQ ID NO: 4, 14, 24, 34, 44, 54, 64 or 74;
   (e) the CDRL2 has an amino acid sequence as set forth in SEQ ID NO: 5, 15, 25, 35, 45, 55, 65 or 75; and
   (f) the CDRL3 has an amino acid sequence as set forth in SEQ ID NO: 6, 16, 26, 36, 46, 56, 66 or 76.

2. The antibody according to claim 1, wherein:

   (1) the CDRH1 has an amino acid sequence as set forth in SEQ ID NO: 1, the CDRH2 has an amino acid sequence as set forth in SEQ ID NO: 2, the CDRH3 has an amino acid sequence as set forth in SEQ ID NO: 3, the CDRL1 has an amino acid sequence as set forth in SEQ ID NO: 4, the CDRL2 has an amino acid sequence as set forth in SEQ ID NO: 5, and CDRL3 has an amino acid sequence as set forth in SEQ ID NO: 6; or
   (2) the CDRH1 has an amino acid sequence as set forth in SEQ ID NO: 11, the CDRH2 has an amino acid sequence as set forth in SEQ ID NO: 12, the CDRH3 has an amino acid sequence as set forth in SEQ ID NO: 13, the CDRL1 has an amino acid sequence as set forth in SEQ ID NO: 14, the CDRL2 has an amino acid sequence as set forth in SEQ ID NO: 15, and CDRL3 has an amino acid sequence as set forth in SEQ ID NO: 16; or
   (3) the CDRH1 has an amino acid sequence as set forth in SEQ ID NO: 21, the CDRH2 has an amino acid sequence as set forth in SEQ ID NO: 22, the CDRH3 has an amino acid sequence as set forth in SEQ ID NO: 23, the CDRL1 has an amino acid sequence as set forth in SEQ ID NO: 24, the CDRL2 has an amino acid sequence as set forth in SEQ ID NO: 25, and CDRL3 has an amino acid sequence as set forth in SEQ ID NO: 26; or
   (4) the CDRH1 has an amino acid sequence as set forth in SEQ ID NO: 31, the CDRH2 has an amino acid sequence as set forth in SEQ ID NO: 32, the CDRH3 has an amino acid sequence as set forth in SEQ ID NO: 33, the CDRL1 has an amino acid sequence as set forth in SEQ ID NO: 34, the CDRL2 has an amino acid sequence as set forth in SEQ ID NO: 35, and CDRL3 has an amino acid sequence as set forth in SEQ ID NO: 36; or
   (5) the CDRH1 has an amino acid sequence as set forth in SEQ ID NO: 41, the CDRH2 has an amino acid sequence as set forth in SEQ ID NO: 42, the CDRH3 has an amino acid sequence as set forth in SEQ ID NO: 43, the CDRL1 has an amino acid sequence as set forth in SEQ ID NO: 44, the CDRL2 has an amino acid sequence as set forth in SEQ ID NO: 45, and CDRL3 has an amino acid sequence as set forth in SEQ ID NO: 46; or
   (6) the CDRH1 has an amino acid sequence as set forth in SEQ ID NO: 51, the CDRH2 has an amino acid sequence as set forth in SEQ ID NO: 52, the CDRH3 has an amino acid sequence as set forth in SEQ ID NO: 53, the CDRL1 has an amino acid sequence as set forth in SEQ ID NO: 54, the CDRL2 has an amino acid sequence as set forth in SEQ ID NO: 55, and CDRL3 has an amino acid sequence as set forth in SEQ ID NO: 56; or

(7) the CDRH1 has an amino acid sequence as set forth in SEQ ID NO: 61, the CDRH2 has an amino acid sequence as set forth in SEQ ID NO: 62, the CDRH3 has an amino acid sequence as set forth in SEQ ID NO: 63, the CDRL1 has an amino acid sequence as set forth in SEQ ID NO: 64, the CDRL2 has an amino acid sequence as set forth in SEQ ID NO: 65, and CDRL3 has an amino acid sequence as set forth in SEQ ID NO: 66; or

(8) the CDRH1 has an amino acid sequence as set forth in SEQ ID NO: 71, the CDRH2 has an amino acid sequence as set forth in SEQ ID NO: 72, the CDRH3 has an amino acid sequence as set forth in SEQ ID NO: 73, the CDRL1 has an amino acid sequence as set forth in SEQ ID NO: 74, the CDRL2 has an amino acid sequence as set forth in SEQ ID NO: 75, and CDRL3 has an amino acid sequence as set forth in SEQ ID NO: 76.

3. The antibody according to claim 1, wherein the heavy chain variable region comprises an amino acid sequence as set forth in SEQ ID NO: 7, 17, 27, 37, 47, 57, 67 or 77 or an amino acid sequence having at least 90%, at least 95%, at least 96%, at least 97%, at least 98%, at least 99% or at least 99.5% sequence identity with the amino acid sequence as set forth in SEQ ID NO: 7, 17, 27, 37, 47, 57, 67 or 77.

4. The antibody according to claim 3, wherein the light chain variable region comprises an amino acid sequence as set forth in SEQ ID NO: 8, 18, 28, 38, 48, 58, 68 or 78 or an amino acid sequence having at least 90%, at least 95%, at least 96%, at least 97%, at least 98%, at least 99% or at least 99.5% sequence identity with the amino acid sequence as set forth in SEQ ID NO: 8, 18, 28, 38, 48, 58, 68 or 78.

5. The antibody according to any one of claims 1-4, wherein the heavy chain variable region and/or the light chain variable region is/are a part of a single-chain variable fragment scFv, a F(ab')$_2$ fragment, a Fab or Fab' fragment, a bivalent antibody, a trivalent antibody, a tetravalent antibody or a monoclonal antibody.

6. A pharmaceutical composition, comprising the antibody according to any one of claims 1-5 and a pharmaceutically acceptable carrier.

7. A nucleic acid molecule, comprising a nucleotide sequence encoding the antibody according to any one of claims 1-5.

8. The nucleic acid molecule according to claim 7, comprising a heavy chain variable region coding sequence as set forth in SEQ ID NO: 9, 19, 29, 39, 49, 59, 69 or 79 or a nucleotide sequence having at least 90%, at least 95%, at least 96%, at least 97%, at least 98%, at least 99% or at least 99.5% sequence identity with the heavy chain variable region coding sequence as set forth in SEQ ID NO: 9, 19, 29, 39, 49, 59, 69 or 79, and/or a light chain variable region coding sequence as set forth in SEQ ID NO: 10, 20, 30, 40, 50, 60, 70 or 80 or a nucleotide sequence having at least 90%, at least 95%, at least 96%, at least 97%, at least 98%, at least 99% or at least 99.5% sequence identity with the light chain variable region coding sequence as set forth in SEQ ID NO: 10, 20, 30, 40, 50, 60, 70 or 80.

9. Use of the antibody according to any one of claims 1-5 or the pharmaceutical composition according to claim 6 or the nucleic acid molecule according to any one of claims 7-8 in manufacturing a drug for treating colorectal carcinoma.

10. Use of the antibody according to any one of claims 1-5 or the pharmaceutical composition according to claim 6 or the nucleic acid molecule according to any one of claims 7-8 in manufacturing a kit for diagnosing head and neck squamous cell carcinoma, renal carcinoma, prostate carcinoma, and colorectal carcinoma.

Fig. 1

| Grade | Time | Concentration | Area | Height |
|-------|------|--------------|------|--------|
| 1 | 11. 728 | 2. 022 | 407781 | 27688 |
| 2 | 12. 493 | 97. 08 | 19573235 | 1430290 |
| 3 | 21. 137 | 0. 9024 | 181965 | 16708 |
| Total | | 100 | 20162981 | 1474686 |

Fig. 2

1: Anti-HMMW-1 antibody    2: Anti-HMMW-2 antibody
3: Anti-HMMW-3 antibody    4: Anti-HMMW-4 antibody
5: Anti-HMMW-5 antibody    6: Anti-HMMW-6 antibody
7: Anti-HMMW-7 antibody    8: Anti-HMMW-8 antibody

Fig. 3

Fig. 4

Colorectal carcinoma cells HCT116

Fig. 5

Colorectal carcinoma cells HCT116

Fig. 6

<div style="text-align:center">**INTERNATIONAL SEARCH REPORT**</div>

| International application No. |
|---|
| **PCT/CN2022/125196** |

**A.    CLASSIFICATION OF SUBJECT MATTER**

C07K 16/18(2006.01)i;  C12N 15/13(2006.01)i;  A61K 39/395(2006.01)i;  A61P 35/00(2006.01)i

According to International Patent Classification (IPC) or to both national classification and IPC

**B.    FIELDS SEARCHED**

Minimum documentation searched (classification system followed by classification symbols)

C07K C12N A61K A61P

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI, 中国专利生物序列检索系统 China Patent Biological Sequence Search System, Genbank, EMBL, STN: 南京安吉, 抗体, HMMW, SEQ ID NOs: 1-80

**C.    DOCUMENTS CONSIDERED TO BE RELEVANT**

| Category* | Citation of document, with indication, where appropriate, of the relevant passages | Relevant to claim No. |
|---|---|---|
| PX | CN 114014928 A (NANJING ANJI BIOTECHNOLOGY CO., LTD.) 08 February 2022 (2022-02-08)<br>      claims 1-10 | 1-10 |
| A | CN 112442116 A (NANJING ANJI BIOTECHNOLOGY CO., LTD.) 05 March 2021 (2021-03-05)<br>      Entire document, in particular abstract, claims 1-10, and embodiments | 1-10 |
| A | CN 109890846 A (MERCK SHARP & DOHME CORP.; MERCK SHARP & DOHME LTD.) 14 June 2019 (2019-06-14)<br>      description, paragraphs 237-240 | 1-10 |
| A | CN 110407941 A (SHANGHAI EPIMAB BIOTHERAPEUTICS CO., LTD.) 05 November 2019 (2019-11-05)<br>      claims 1-11 | 1-10 |
| A | JP 2001057888 A (DAI ICHI SEIYAKU CO., LTD.) 06 March 2001 (2001-03-06)<br>      abstract, and claims 1-18 | 1-10 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 November 2022** | **12 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**EP 4 198 054 A1**

## INTERNATIONAL SEARCH REPORT

International application No.

**PCT/CN2022/125196**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1]   The actually submitted sequence listing is an XML file in Standard ST.26.

Form PCT/ISA/210 (continuation of first sheet) (January 2015)

**INTERNATIONAL SEARCH REPORT**
**Information on patent family members**

International application No.

**PCT/CN2022/125196**

| Patent document cited in search report | | | Publication date (day/month/year) | Patent family member(s) | | | Publication date (day/month/year) |
|---|---|---|---|---|---|---|---|
| CN | 114014928 | A | 08 February 2022 | None | | | |
| CN | 112442116 | A | 05 March 2021 | None | | | |
| CN | 109890846 | A | 14 June 2019 | US | 2019300618 | A1 | 03 October 2019 |
| | | | | AU | 2017331380 | A1 | 28 March 2019 |
| | | | | JP | 2020114241 | A | 30 July 2020 |
| | | | | PE | 20190737 | A1 | 23 May 2019 |
| | | | | GE | P20217263 | B | 10 June 2021 |
| | | | | US | 2020131272 | A1 | 30 April 2020 |
| | | | | TN | 2019000075 | A1 | 15 July 2020 |
| | | | | NI | 201900024 | A | 27 May 2019 |
| | | | | JP | 2022046684 | A | 23 March 2022 |
| | | | | MA | 46272 | A | 31 July 2019 |
| | | | | KR | 20190053949 | A | 20 May 2019 |
| | | | | CL | 2021000088 | A1 | 02 July 2021 |
| | | | | JO | P20190055 | A1 | 16 June 2017 |
| | | | | DO | P2019000077 | A | 30 June 2019 |
| | | | | CO | 2019002671 | A2 | 29 March 2019 |
| | | | | IL | 265434 | A | 30 May 2019 |
| | | | | JP | 2019535235 | A | 12 December 2019 |
| | | | | CA | 3036573 | A1 | 29 March 2018 |
| | | | | BR | 112019005726 | A2 | 23 June 2020 |
| | | | | WO | 2018058022 | A1 | 29 March 2018 |
| | | | | MX | 2019003405 | A | 30 May 2019 |
| | | | | US | 2018086841 | A1 | 29 March 2018 |
| | | | | EC | SP19020740 | A | 30 April 2019 |
| | | | | KR | 20210040465 | A | 13 April 2021 |
| | | | | AR | 109715 | A1 | 16 January 2019 |
| | | | | PH | 12019500628 | A1 | 05 August 2019 |
| | | | | EP | 3515941 | A1 | 31 July 2019 |
| | | | | CR | 20190140 | A | 17 May 2019 |
| | | | | TW | 201815824 | A | 01 May 2018 |
| | | | | AU | 2021200984 | A1 | 11 March 2021 |
| | | | | CL | 2019000779 | A1 | 19 July 2019 |
| CN | 110407941 | A | 05 November 2019 | WO | 2021056610 | A1 | 01 April 2021 |
| | | | | EP | 4077386 | A1 | 26 October 2022 |
| | | | | TW | 202126691 | A | 16 July 2021 |
| | | | | CN | 114729038 | A | 08 July 2022 |
| JP | 2001057888 | A | 06 March 2001 | None | | | |

Form PCT/ISA/210 (patent family annex) (January 2015)